# NEUE EUROPÄISCHE PATENTSCHRIFT

(11) **EP 1 155 139 B2**
(45) Veröffentlichungstag und Bekanntmachung des Hinweises auf die Entscheidung über den Einspruch: **25.02.2009**
(45) Hinweis auf die Patenterteilung: 09.04.2003
(21) Anmeldenummer: 00906363.7
(22) Anmeldetag: 21.02.2000
(51) Int. Cl.: C12P 13/06, C12N 1/21

(54) **VERFAHREN ZUR MIKROBIELLEN HERSTELLUNG VON L-VALIN**
METHOD FOR MICROBIALLY PRODUCING L-VALINE
PROCEDE POUR LA PRODUCTION MICROBIENNE DE L-VALINE

(30) Priorität: 22.02.1999 DE 19907567
(43) Veröffentlichungstag der Anmeldung: 21.11.2001
(73) Patentinhaber: Forschungszentrum Jülich GmbH, 52425 Jülich (DE)
(72) Erfinder: EGGELING, Lothar, D-52428 Jülich (DE); SAHM, Hermann, D-52428 Jülich (DE)
(86) Internationale Anmeldenummer: PCT/EP2000/001405
(87) Internationale Veröffentlichungsnummer: WO 2000/050624

(56) Entgegenhaltungen:
- EP-A- 0 136 359
- EP-A- 0 356 739
- EP-A- 0 436 886
- EP-A- 0 872 547
- KEILHAUER C ET AL: "ISOLEUCINE SYNTHESIS IN CORYNEBACTERIUM GLUTAMICUM: MOLECULAR ANALYSIS OF THE ILVB-ILVN-ILVC OPERON" JOURNAL OF BACTERIOLOGY,US,WASHINGTON, DC, Bd. 175, Nr. 17, 1. September 1993 (1993-09-01), Seiten 5595-5603, XP000611312 ISSN: 0021-9193 in der Anmeldung erwähnt
- LAWTHER R ET AL: "The complete nucleotide sequence of the ilvGMEDA operon of E. coli K-12" NUCLEIC ACIDS RESEARCH, Bd. 15, Nr. 8, 1987, Seiten 2137-2155, XP002138603 OXFORD GB
- VELASCO JUAN A ET AL: "Cloning of the dihydroxyacid dehydratase-encoding gene (ILV3) from Saccharomyces cerevisiae." GENE (AMSTERDAM) 1993, Bd. 137, Nr. 2, 1993, Seiten 179-185, XP002138604 ISSN: 0378-1119
- DATABASE BIOSIS [Online] BIOSCIENCES INFORMATION SERVICE, PHILADELPHIA, PA, US1993 MILLER STANLEY L ET AL: "Prebiotic syntheses of vitamin coenzymes: II. Pantoic acid, pantothenic acid, and the composition of coenzyme A." Database accession no. PREV199395129991 XP002138606 & JOURNAL OF MOLECULAR EVOLUTION 1993, Bd. 36, Nr. 4, 1993, Seiten 308-314, ISSN: 0022-2844
- SAHM HERMANN ET AL: "D-pantothenate synthesis in Corynebacterium glutamicum and use of panBC and genes encoding L-valine synthesis for D-pantothenate overproduction." APPLIED AND ENVIRONMENTAL MICROBIOLOGY MAY, 1999, Bd. 65, Nr. 5, Mai 1999 (1999-05), Seiten 1973-1979, XP002138605 ISSN: 0099-2240

## Beschreibung

Die vorliegende Erfindung bezieht sich auf ein Verfahren zur mikrobiellen Herstellung von L-Valin nach Anspruch 1 bis 11 sowie auf im Verfahren einsetzbare, transformierte Mikroorganismen nach Anspruch 12 bis 15.

Die Aminosäure L-Valin stellt ein kommerziell bedeutendes Produkt dar, das in der Tierernährung, der Humanernährung und der Medizin Anwendung findet. Es besteht daher ein allgemeines Interesse daran, verbesserte Verfahren zur Herstellung von L-Valin bereitzustellen.

Valin kann durch chemische Synthese oder biotechnologisch durch Fermentation geeigneter Mikroorganismen in geeigneten Nährlösungen hergestellt werden. Der Vorteil der biotechnologischen Herstellung durch Mikroorganismen liegt in der Bildung der korrekten stereo-isomeren Form, nämlich der L-Form von Valin frei von D-Valin.

Verschiedene Arten von Bakterien, wie z. B. Escherichia coli, Serratia marcescens, Corynebacterium glutamicum, Brevibacterium flavum oder Brevibacterium lactofermentum können in einer Nährlösung, die Glucose enthält, L-Valin produzieren. US 5 658 766 zeigt, daß bei Escherichia coli durch Mutation in derAminoacyl-tRNA-Synthetase eine gesteigerte Bildung von L-Valin erreicht werden kann. WO 96 06926 zeigt weiterhin, dass durch eine Liponsäure-Auxotrophie eine Steigerung der L-Valinbildung mit Escherichia coli erreicht werden kann. EP 0 694 614 A1 beschreibt Stämme von Escherichia coli, die Resistenzen gegenüber α-Ketobuttersäure tragen und in einer Nährlösung, die Glucose enthält, L-Valin, L-Isoleucin oder L-Leucin produzieren.

In EP 0 477 000 wird gezeigt, dass durch Mutagenese von Corynebacterium oder Brevibacterium und Selektion auf Valin-Resistenz die L-Valinbildung verbessert werden kann. In derselben EP-Schrift wird auch gezeigt, dass durch Selektion von Corynebacterium oder Brevibacterium auf Resistenz gegenüber verschiedenen Pyruvat-Analoga, wie β-Fluoropyruvat, β-Chloropyruvat, β-Mercaptopyruvat oder Trimethylpyruvat eine verbesserte L-Valinbildung erreicht werden kann. Durch Nakayama et al. (Nakayama et al., 1961, J. Gen. Appl. Microbiol. Jpn) ist bekannt, dass durch ungerichtete Mutationen eingeführte Auxotrophien zu verbesserter L-Valinakkumulation führen können.

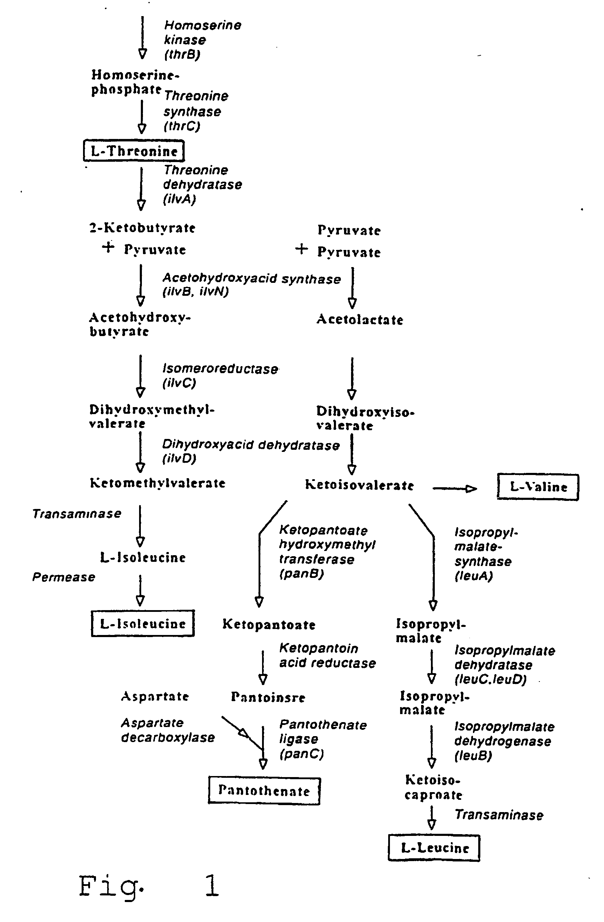

In EP 0 356 739 A1 wird darüber hinaus gezeigt, dass bei Amplifikation des für die Acetohydroxysäuresynthase (ilvBN, siehe auch Figur 1) kodierenden DNA-Bereichs mittels des Plasmids pAJ220V3 die Bildung von L-Valin verbessert wird.

EP-A-872547 offenbart, dass bei *Escherichia* Mikroorganismen, die Produktion von L-Valin durch eine Modifikation des H⁺ATP-Gens erhöht werden kann. Das Gen kann auch das IlvA-Gen einschließen.

Journal of Bacteriology, 1993, Band 175, Nr. 17, Seiten 5595-5603 offenbart, dass Acetohydroxysäuresynthase und Isomeroreductase (ilvC) Katalysatoren sind für nacheinander folgende Reaktionen im Weg zu u. a. Valin in Co*rynebacterium glutamicum*. Acetohydroxysäuresynthase wird durch zwei Gene kodiert, ilvB und ilvN.

Nucleic Acids Research, 1987, Band 15, Nr. 5, Seiten 2137-2155 offenbart die Sequenzierung des IlvGMEDA Operons von *E.coli*, das 5 Gene enthält, die für 4 von den 5 Enzymen kodieren, die für die Biosynthese von L-Valin notwendig sind.

Gene, 1993, Band 137, Seiten 179-185 offenbart die Sequenzierung vom ilv3-Gen in *Saccharomyces cerevisiae*, das für Dihydroxysäurehydratase-Biosynthese notwendig ist.

Die japanische Schrift Tokkai Hei 8-89249 offenbart eine aus coryneformen Bakterien stammende DNS, die für Dihydroxysäure-Dehydratase kodiert und die für die Produktion von L-Valin verwendet werden kann.

Die japanische Schrift Tokkai Hei 5-344 893 zeigt, dass die Produktion von L-Valin durch die Verwendung von Plasmiden, welche das Gen von AcetohydroxysäureSynthase tragen gesteigert werden kann.

Die Veröffentlichung "Isoleucine Synthesis in *Corynebacterium glutamicum*: Molecular Analysis of ilvB - ilvN - ilvC Operon" von C. Keilhauer et al., Journal of Bacteriology, Sept. 1993, p. 5595-5603 offenbart Strukturanalysen des ilvBNC Operons.

Die EP 1006 189 A2 offenbart ein Verfahren zur Herstellung von D-Pantothensäure bei dem die Gene panB und panC einzeln oder kombiniert miteinander verstärkt, insbesondere überexprimiert werden. Fakultativ kann eine Defektmutation von ilvA vorgenommen oder eine Verstärkung bzw. Überexpression der Gene ilvBN, ilvD oder ilvC vorgenommen werden.

Es ist Aufgabe der vorliegenden Erfindung, neue Grundlagen zur mikrobiellen Herstellung von L-Valin, insbesondere mit Hilfe coryneformer Bakterien, bereitzustellen.

Diese Aufgabe wird erfindungsgemäß dadurch gelöst, dass die Dihydroxysäuredehydratase- (ilvD-) Aktivität und/oder die ilvD-Genexpression in einem Mikroorganismus verstärkt wird, wobei die Aktivität eines oder mehrerer, an der Synthese von D-Pantothenat spezifisch beteiligter Enzyme abgeschwächt oder ausgeschaltet ist. In Kombination damit wird die Acetohydroxysäuresynthase- (ilvBN-) und Isomeroreduktase-(ilvC-) Aktivität und/oder die ilvBNC-Genexpression in einem Mikroorganismus verstärkt. Für die erfindungsgemäßen Verfahren können zusätzlich Mikroorganismen zum Einsatz kommen, in denen die Aktivität zumindest eines Enzyms, das an einem Stoffwechselweg beteiligt ist, der die L-Valinbildung herabsetzt, abgeschwächt oder ausgeschaltet ist. So werden in den erfindungsgemäßen Verfahren vorzugsweise Mikroorganismen mit einer Defektmutation im Threonindehydratase- (ilvA-) Gen und/oder mit einer Defektmutation in einem oder mehreren Genen der Pantothenatsynthese eingesetzt.

Mit den Begriffen "Valin" oder "L-Valin" ist im Sinne der beanspruchten Erfindung nicht nur die Freie Säure, sondern auch das Salz davon, wie z. B. das Calcium-, Natrium-, Ammonium- oder Kaliumsalz, gemeint.

Der Begriff "Verstärkung" beschreibt die Erhöhung der intrazellulären Aktivität der genannten Enzyme ilvD, ilvB, ilvN und ilvC. Zur Erhöhung der Enzymaktivität wird insbesondere die endogene Aktivität im Mikroorganismus erhöht. Eine Erhöhung der Enzymaktivität kann beispielsweise erreicht werden, in dem durch Veränderung des katalytischen Zentrums ein erhöhter Substratumsatz erfolgt oder in dem die Wirkung von Enzyminhibitoren aufgehoben wird. Auch kann eine erhöhte Enzymaktivität durch Erhöhung der Enzymsynthese, beispielsweise durch Genamplifikation oder durch Ausschaltung von Faktoren, die die Enzymbiosynthese reprimieren, hervorgerufen werden. Die endogene Enzymaktivität wird erfindungsgemäß vorzugsweise durch Mutation des entsprechenden endogenen Gens erhöht. Derartige Mutationen können entweder nach klassischen Methoden ungerichtet erzeugt werden, wie beispielsweise UV-Bestrahlung oder mutationsauslösenden Chemikalien, oder gezielt mittels gentechnologischer Methoden, wie Deletion(en), Insertion(en) und/oder Nukleotidaustausch(e).

Die Verstärkung der Genexpression erfolgt erfindungsgemäß vorzugsweise durch Erhöhung der Genkopienzahl. Dazu wird das Gen bzw. werden die Gene in ein Genkonstrukt bzw. in einen Vektor eingebaut, der vorzugsweise den Genen zugeordnete regulatorische Gensequenzen enthält, insbesondere solche, die die Genexpression verstärken. Anschließend wird ein Mikroorganismus, vorzugsweise Corynebacterium glutamicum, mit den entsprechenden Genkonstrukten transformiert.

Es wurde festgestellt, daß durch verstärkte Expression des Valinbiosynthesegens ilvD aus Corynebacterium glutamicum, welches für das Enzym Dihydroxysäuredehydratase kodiert, in verbesserter Weise L-Valin produziert wird. Erfindungsgemäss bewirken neben der verstärkten Expression dieses Gens auch die verstärkte Expression der ilvBN-Gene, die für das Enzym Acetohydroxysäuresynthase kodieren, und des ilvC-Gens, das für das Enzym Isomeroreduktase kodiert, in Corynebacterium glutamicum eine verbesserte L-Valinbildung. Eine weitere Verbesserung der L-Valinbildung wird durch Überexpression aller genannten Gene in Corynebacterium glutamicum erreicht. Die Gene oder Genkonstrukte können im Wirtsorganismus entweder in Plasmiden mit unterschiedlicher Kopienzahl vorliegen oder im Chromosom integriert und amplifiziert sein.

Eine weitere Erhöhung der Genexpression kann - alternativ oder kombiniert mit einer Erhöhung der Genkopienzahl - durch Verstärkung regulatorischer Faktoren, die die Genexpression positiv beeinflussen, bewirkt werden. So kann eine Verstärkung regulatorischer Elemente auf Transkriptionsebene erfolgen, indem insbesondere verstärkte Transkriptionssignale verwendet werden. Auch kann die Promotor- und Regulationsregion, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich die Expression im Verlaufe der fermentativen L-Valinbildung zu steigern. Daneben ist aber auch eine Verstärkung der Translation möglich, indem beispielsweise die Stabilität der m-RNA verbessert wird. Desweiteren können Gene verwendet werden, die für das entsprechende Enzym mit einer hohen Aktivität kodieren. Alternativ kann weiterhin eine Überexpression der betreffenden Gene durch Veränderung der Medienzusammensetzung und Kulturführung erreicht werden. Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der Europäischen Patentschrift EP 0 472 869, im US Patent 4,601,893, bei Schwarzer und Pühler (Bio/Technology 9, 84-87 (1991), bei Reinscheid et al. (Applied and Environmental Microbiology 60,126-132 (1994)), bei LaBarre et al. (Journal of Bacteriology 175, 1001-1007 (1993)) und in der Patentanmeldung WO 96/15246.

Für eine Verstärkung der Genexpression sind ebenso alle denkbaren Kombinationen der oben genannten Maßnahmen möglich.

Mikroorganismen, die im erfindungsgemäßen Verfahren einsetzbar sind, können L-Valin aus Glucose, Saccharose, Lactose, Fructose, Maltose, Melasse, Stärke, Cellulose oder aus Glycerin und Ethanol herstellen. Es kann sich um Gram-positive Bakterien z. B. der Gattung Bacillus oder um coryneforme Bakterien der bereits erwähnten Gattung Corynebacterium oder auch um Arthrobacter handeln. Bei der Gattung Corynebacterium wurde insbesondere bereits die Art Corynebacterium glutamicum genannt, die in der Fachwelt für ihre Fähigkeit bekannt ist Aminosäuren zu bilden. Zu dieser Art gehören Wildtypstämme, wie z. B. Corynebacterium glutamicum ATCC13032, Brevibacterium flavum ATCC14067, Brevibacterium lactofermentum ATCC13869, Brevibacterium thiogenitalis ATCC19240, Corynebacterium melassecola ATCC17965 und andere.

Zur Isolierung des Gens ilvD von Corynebacterium glutamicum oder anderer Gene wird zunächst eine Genbank angelegt. Das Anlegen von Genbanken ist in allgemein bekannten Lehrbüchern und Handbüchern niedergeschrieben. Als Beispiel seien das Lehrbuch von Winnacker: Gene und Klone, Eine Einführung in die Gentechnologie (Verlag Chemie, Weinheim, Deutschland, 1990) oder das Handbuch von Sambrook et al.: Molecular Cloning, A Laboratory Manual (Cold Spring Harbor Laboratory Press, 1989) genannt. Eine bekannte Genbank ist die des E. coli K-12 Stammes W3110, die von Kohara et al. (Cell 50, 495 - 508 (1987)) die in λ-Vektoren angelegt wurde. Bathe et al. (Molecular and General Genetics, 252:255-265, 1996) beschreiben eine Genbank von Corynebacterium glutamicum ATCC13032, die mit Hilfe des Cosmidvektors SuperCos I (Wahl et al., 1987, Proceedings of the National Academy of Sciences USA, 84:2160-2164) im E.coli K-12 NM554 (Raleigh et al., 1988, Nucleic Acids Research 16:1563-1575) angelegt wurde. Zur Herstellung einer Genbank von Corynebacterium glutamicum in Escherichia coli können auch Plasmide wie pBR322 (Bolivar, Life Sciences, 25, 807-818 (1979)) oder pUC19 (Norrander et al., 1983, Gene, 26: 101-106) verwendet werden. Zur Herstellung einer Genbank von Corynebacterium glutamicum in Corynebacterium glutamicum können Plasmide wie pJC1 (Cremer et al., Mol. Gen. Genet. (1990) 220: 3221-3229) oder pECM2 (Jäger et al., J. Bacteriol. (1992) 174: 5462-5465) verwendet werden. Als Wirte eignen sich besonders solche Bakterien-Stämme, die restriktions- und rekombinationsdefekt sind. Ein Beispiel hierfür ist der Stamm Escherichia coli DH5α*mcr*, der von Grant et al. (Proceedings of the National Academy of Sciences USA, 87 (1990) 4645-4649) beschrieben wurde, oder der Stamm Corynebacterium glutamicum R127, der von Liebl et al. isoliert wurde (FEMS Lett (1989) 65: 299-304).

Die Genbank wird anschließend in einen Indikatorstamm durch Transformation (Hanahan, Journal of Molecular Biology 166, 557-580, 1983) oder Elektroporation (Tauch et.al., 1994, FEMS Microbiological Letters, 123: 343-347) eingebaut. Der Indikatorstamm zeichnet sich dadurch aus, dass er eine Mutation in dem interessierenden Gen besitzt, die einen detektierbaren Phänotyp, z.B. eine Auxotrophie, hervorruft. Die Indikatorstämme bzw. Mutanten sind aus publizierten Quellen oder Stammsamrnlungen erhältlich oder müssen gegebenfalls selbst hergestellt werden. Im Rahmen der vorliegenden Erfindung ist die Corynebacterium glutamicum Mutante R127/7 isoliert worden, die in dem für die Dihydroxysäuredehydratase kodierendem ilvD-Gen defekt ist. Nach Transformation des Indikatorstammes, wie z.B. der ilvD-Mutante R127/7, mit einem rekombinanten Plasmid, welches das interessierende Gen, wie z.B. das ilvD-Gen trägt und Expression desselben, wird der Indikatorstamm bezüglich der entsprechenden Eigenschaft, wie z.B. der L-Valin-Bedürftigkeit, prototroph.

Das dergestalt isolierte Gen bzw. DNA-Fragment kann durch Bestimmung der Sequenz, wie z.B. bei Sanger et al. (Proceedings of the National of Sciences of the United States of America USA, 74:5463-5467, 1977) beschrieben, charakterisiert werden. Anschließend kann der Grad an Identität zu bekannten Genen, die in Datenbanken wie z.B. der GenBank (Benson et el., 1998, Nuleic Acids Research, 26:1-7) enthalten sind, mit publizierten Methoden analysiert werden (Altschul et al., 1990, Journal of Molecular Biology 215:403-410).

Auf diese Weise wurde die für das Gen ilvD kodierende DNA-Sequenz von Corynebacterium glutamicum erhalten, die als SEQ ID NO 1 Bestandteil der vorliegenden Erfindung ist. Weiterhin wurden aus der vorliegenden DNA-Sequenz mit den oben beschriebenen Methoden die Aminosäuresequenzen der entsprechenden Enzyme abgeleitet. In SEQ ID NO 2 ist die sich ergebende Aminosäuresequenz des ilvD-Genproduktes, nämlich der Dihydroxysäuredehydratase, dargestellt.

Das dergestalt charakterisierte Gen kann anschließend einzeln oder in Kombination mit anderen in einem geeigneten Mikroorganismus zur Expression gebracht werden. Eine bekannte Methode, Gene zu exprimieren bzw. überzuexprimieren, besteht darin, diese mit Hilfe von Plasmidvektoren zu amplifizieren, die überdies mit Expressionssignalen ausgestattet sein können. Als Plasmidvektoren kommen solche in Frage, die in den entsprechenden Mikroorganismen replizieren können. Für Corynebacterium glutamicum kommen z.B. die Vektoren pEKEx1 (Eikmanns et al., Gene 102:93-98 (1991)) oder pZ8-1 (Europäische Patentschrift 0 375 889) oder pEKEx2 (Eikmanns et al. Microbiology 140: 1817-1828 (1994) oder pECM2 (Jäger et al. Journal of Bacteriology 174(16): 5462-5465 (1992)) in Frage. Beispiele für derartige Plasmide sind pJC1ilvD, pECM3ilvBNCD, und pJC1ilvBNCD. Diese Plasmide sind Escherichia coli/Corynebacterium glutamicum Pendelvektor die das Gen ilvD bzw. das Gen ilvD zusammen mit den Genen ilvB, ilvN, und ilvC tragen.

Die Erfinder haben weiterhin gefunden, dass sich die Verstärkung des Gens einzeln oder in Kombination mit den Genen ilvB, ilvN und ilvC in solchen Mikroorganismen vorteilhaft auswirkt, die eine reduzierte Synthese der Aminosäure L-Isoleucin aufweisen. Diese reduzierte Synthese kann durch Deletion des ilvA-Gens erreicht werden, das für das für die L-Isoleucinsynthese spezifische Enzym Threonindehydratase kodiert.

Die Deletion kann durch gerichtete rekombinante DNA-Techniken erfolgen. Mit Hilfe dieser Methoden kann zum Beispiel das für die Threonindehydratase kodierende ilvA-Gen im Chromosom deletiert werden. Geeignete Methoden dazu sind bei Schäfer et al. (Gene (1994) 145: 69-73) oder auch Link et al. (Journal of Bacteriology (1998) 179: 6228-6237) beschrieben. Auch können nur Teile des Gens deletiert werden, oder auch mutierte Fragmente des Threonindehydratasegens ausgetauscht werden. Durch Deletion wird so ein Verlust der Threonindehydrataseaktivität erreicht. Ein Beispiel für eine derartige Mutante ist der Corynebacterium glutamicum Stamm ATCC13032ΔilvA, der eine Deletion im ilvA-Gen trägt.

Die Erfinder haben weiterhin gefunden, dass sich die Verstärkung der Gene ilvD, ilvB, ilvN und ilvC in einer weiteren Kombination mit der reduzierten Synthese von D-Pantothenat, vorzugsweise in Kombination mit weiterer Deletion des ilvA-Gens, in Mikroorganismen vorteilhaft auf die L-Valinbildung auswirkt, so zum Beispiel durch Deletionen im panB- und panC-Gen. Die reduzierte D-Pantothenatsynthese kann durch Abschwächung oder Ausschaltung der entsprechenden Biosyntheseenzyme bzw. Ihrer Aktivitäten erreicht werden. Hierfür kommen zum Beispiel die Enzyme Ketopantoathydroxymethyltransferase (EC 2.1.2.11), Ketopantoatreduktase, Pantothenatligase (EC 6.3.2.1) und die Aspartatdecarboxylase (EC 4.1.1.11) in Frage. Eine Möglichkeit, Enzyme und deren Aktivitäten auszuschalten oder abzuschwächen, sind Mutageneseverfahren.

Hierzu gehören ungerichtete Verfahren, die chemische Reagenzien, wie z.B. N-methyl-N-nitro-N-nitrosoguanidin, oder auch UV-Bestrahlung zur Mutagenese benutzen, mit anschließender Suche der gewünschten Mikroorganismen auf Bedürftigkeit für D-Pantothenat. Verfahren zur Mutationsauslösung und Mutantensuche sind allgemein bekannt und können unter anderem bei Miller (A Short Course in Bacterial Genetics, A Laboratory Manual and Handbook for Escherichia coli and Related Bacteria (Cold Spring Harbor Laboratory Press, 1992)) oder im Handbuch "Manual of Methods for General Bacteriology" der American Society for Bacteriology (Washington D.C., USA, 1981) nachgelesen werden.

Weiterhin gehören hierzu gerichtete rekombinante DNA-Techniken. Mit Hilfe dieser Methoden können zum Beispiel die für die Ketopantoathydroxymethyltransferase, Pantothenatligase, Ketopantoinsäurereduktase oder Aspartatdecarboxylase kodierenden Gene panB, panC, panE und panD einzeln oder auch gemeinsam im Chromosom deletiert werden. Geeignete Methoden dazu sind bei Schäfer et al. (Gene (1994) 145: 69-73) oder auch Link et al. (Journal of Bacteriology (1998) 179: 6228-6237) beschrieben. Auch können nur Teile der Gene deletiert werden oder auch mutierte Fragmente der Ketopantoathydroxymethyltransferase, Pantothenatligase, Ketopantoinsäurereduktase und der Aspartatdecarboxylase ausgetauscht werden. Durch Deletion oder Austausch wird so ein Verlust oder eine Reduktion der jeweiligen Enzymaktivität erreicht. Ein Beispiel für eine derartige Mutante ist der Corynebacterium glutamicum Stamm ATCC13032ΔpanBC, der eine Deletion im panBC Operon trägt.

Die erfindungsgemäß hergestellten Mikroorganismen können kontinuierlich oder diskontinuierlich im batch Verfahren (Satzkultivierung) oder im fed batch (Zulaufverfahren) oder repeated fed batch Verfahren (repetitives Zulaufverfahren) zum Zwecke der L-Valin-Produktion kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Mikroorganismen genügen. Beschreibungen von Kulturmedien verschiedenener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate wie z.B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Kaliumdihydrogenphosphat oder Dikaliumhydrogenphosphat oder die entsprechenden Natrium haltigen Salze verwendet werden. Das Kulturmedium muß weiterhin Salze von Metallen enthalten, wie z.B. Magnesiumsulfat oder Eisensulfat, die für das Wachstum notwendig sind. Schließlich können essentielle Wuchsstoffe, wie Aminosäuren und Vitamine, zusätzlich zu den oben genannten Stoffen eingesetzt werden. Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak oder saure Verbindungen wie Phosphorsäure oder Schwefelsäure in geeigneter Weise eingesetzt. Zur Kontrolle der Schaumentwicklung können Antischaummittel, wie z.B. Fettsäurepolyglykolester, eingesetzt werden. Zur Aufrechterhaltung der Stabilität von Plasmiden können dem Medium geeignete selektiv wirkende Stoffe, z.B. Antibiotika, hinzugefügt werden. Um aerobe Bedingungen aufrechtzuerhalten werden Sauerstoff oder Sauerstoff-haltige Gasmischungen, wie z.B. Luft, in die Kultur eingetragen. Die Temperatur der Kultur liegt normalerweise bei 20°C bis 50°C und vorzugsweise bei 25°C bis 45°C. Die Kultur wird solange fortgesetzt, bis sich ein Maximum an L-Valin gebildet hat. Dieses Ziel wird normalerweise innerhalb von 10 Stunden bis 160 Stunden erreicht.

Die Konzentration an gebildetem L-Valin kann mit bekannten Verfahren (Jones und Gilligan (1983) Journal of Chromatography 266: 471-482) bestimmt werden.

Die Erfindung wird anhand der folgenden Ausführungsbeispiele näher erläutert:

### Beispiel 1: Klonierung, Sequenzierung und Expression des für die Dihydroxysäuredehydratase kodierenden ilvD-Gens aus Corynebacterium glutamicum

### 1. Isolierung einer ilvD Mutante von Corynebacterium glutamicum

Der Stamm Corynebacterium glutamicum R127 (Haynes 1989, FEMS Microbiology Letters 61: 329-334) wurde mit N-methyl-N-nitro-N-nitrosoguanidin mutagenisiert (Sambrook et al., Molecular cloning. A laboratory manual (1989) Cold Spring Harbour Laboratory Press). Dazu wurden 5 ml einer über Nacht angezogenen Corynebacterium glutamicum Kultur mit 250 µl N-methyl-N-nitro-N-nitrosoguanidin (5 mg /ml Dimethylformamid) versetzt und 30 Minuten bei 30 °C und 200 Upm inkubiert (Adelberg 1958, Journal of Bacteriology 76: 326). Die Zellen wurden anschließend zweimal mit steriler NaCl-Lösung (0,9 %) gewaschen. Durch Replikaplattierung auf Minimalmediumplatten CGXII mit 15 g/l Agar (Keilhauer et al., Journal of Bacteriology 175: 5595-5603) wurden Mutanten isoliert, die nur bei Zugabe von L-Valin, L-Isoleucin und L-Leucin (je 0,1 g/l) wuchsen.

Die Enzymaktivität der Dihydroxysäuredehydratase wurde im Rohextrakt dieser Mutanten bestimmt. Dazu wurden die Klone in 60 ml LB-Medium kultiviert und in der exponentiellen Wachstumsphase abzentrifugiert. Das Zellpellet wurde einmal mit 0,05 M Kaliumphosphatpuffer gewaschen und im selben Puffer resuspensiert. Der Zellaufschluß erfolgte mittels 10 minütiger Ultraschallbehandlung (Branson-Sonifier W-250, Branson Sonic Power Co, Danbury, USA). Anschließend wurden die Zelltrümmer durch eine 30 minütige Zentrifugation bei 13000 rpm und 4 °C abgetrennt und der Überstand als Rohextrakt in den Enzymtest eingesetzt. Der Reaktionsansatz des Enzymtests enthielt 0,2 ml 0,25 M Tris/HCl, pH 8, 0,05 ml Rohextrakt, und 0,15 ml 65 mM alpha,β-Dihydroxy-β-methylvalerat. Die Testansätze wurden bei 30 °C inkubiert, nach 10, 20 und 30 Minuten wurde je 200 µl Proben genommen und deren Ketomethylvaleratkonzentration mittels HPLC-Analytik bestimmt (Hara et al. 1985, Analytica Chimica Acta 172: 167-173). Wie Tabelle 1 zeigt, weist der Stamm R127/7 keine Dihydroxysäuredehydrataseaktivität auf, wogegen die Isomeroreduktaseund Acetohydroxysäuresynthaseaktivitäten als weitere Enzyme der Synthese der verzweigtkettigen Aminosäuren noch vorhanden sind.

**Tabelle 1**

| Spezifische Aktivitäten (µmol/min und mg Protein) von Valinbiosyntheseenzymen in Corynebacterium glutamicum Stämmen | | | |
|---|---|---|---|
| Stamm | Dihydroxysäure dehydratase | Isomero reduktase | Acetohydroxysäure synthase |
| R127 | 0,003 | 0,05 | 0,07 |
| R127/7 | 0,000 | 0,06 | 0,09 |

### 2. Klonierung des ilvD-Gens von Corynebacterium glutamicum

Chromosomale DNA aus Corynebacterium glutamicum R127 wurde, wie bei Schwarzer und Pühler (Bio/Technology 9 (1990) 84-87) beschrieben, isoliert.

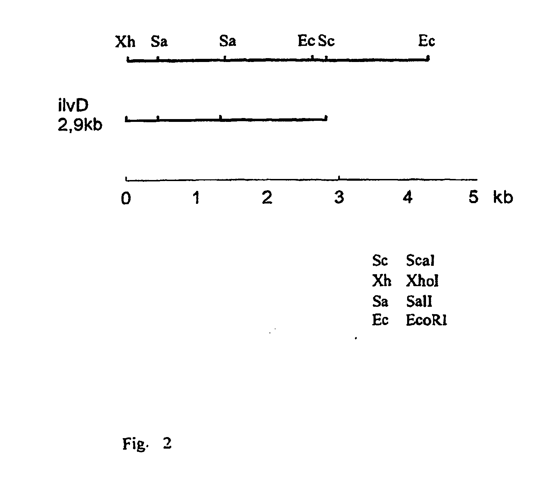

Diese wurde mit dem Restriktionsenzym Sau3A (Boehringer Mannheim) gespalten und durch Saccharose-Dichte-Gradienten-Zentrifugation (Sambrook et al., Molecular cloning. A laboratory manual (1989) Cold Spring Harbour Laboratory Press) aufgetrennt. Die Fraktion mit dem Fragmentgrößenbereich von etwa 6-10 kb wurde zur Ligation mit dem Vektor pJC1 (Cremer et al., Molecular and General Genetics 220 (1990) 478-480) eingesetzt. Der Vektor pJC1 wurde hierzu mit BamHI linearisiert und dephosphoryliert. Fünf ng davon wurden mit 20 ng der genannten Fraktion der chromosomalen DNA ligiert und damit die Mutante R127/7 durch Elektroporation (Haynes und Britz, FEMS Microbiology Letters 61 (1989) 329-334) transformiert. Die Transformanten wurden auf die Fähigkeit getestet, auf CGXII Agarplatten ohne Zugabe der verzweigtkettigen Aminosäuren wachsen zu können. Von über 5000 getesteten Transformanden wuchsen nach Replicaplattierung und zweitägiger Inkubation bei 30°C 8 Klone auf Minmalmediumplatten. Von diesen Klonen wurden Plasmidpräparationen, wie bei Schwarzer et al. (Bio/Technology (1990) 9: 84-87) beschrieben durchgeführt. Restriktionsanalysen der Plasmid-DNA ergaben, daß in allen 8 Klonen dasselbe Plasmid, im Folgendem pRV genannt, enthalten war. Das Plasmid trägt ein Insert von 4,3 kb und wurde durch Retransformation auf seine Fähigkeit die ilvD-Mutante R127/7 zu komplementieren getestet. Durch Subklonierung wurde der für die Komplementation der Mutante R127/7 verantwortliche Bereich auf ein 2,9 ScaI/XhoI-Fragment eingegrenzt (Figur 2).

### 3. Sequenzierung des ilvD-Gens

Die Nukleinsäuresequenz des 2,9 kb ScaI/XhoI-Fragments wurde nach der Dideoxy-Kettenabbruchmethode von Sanger et al. durchgeführt (Proceedings of the National of Sciences of the United States of America USA (1977) 74: 5463-5467). Dabei wurde der Auto-Read Sequencing kit verwendet (Amersham Pharmacia Biotech, Uppsala, Schweden). Die gelelektrophoretische Analyse erfolgte mit dem automatischem Laser-Fluoreszenz Sequenziergerät (A.L.F.) von Amersham Pharmacia Biotech (Uppsala, Schweden). Die erhaltene Nukleotidsequenz wurde mit dem Programmpaket HUSAR (Release 4.0, EMBL, Cambridge, GB) analysiert. Die Nukleotidsequenz ist als ID SEQ NO 1 wiedergegeben. Die Analyse ergab ein offenes Leseraster von 1836 Basenpaaren, das als ilvD-Gen identifiziert wurde und für ein Polypeptid von 612 Aminosäuren kodiert, das als SEQ ID NO 2 wiedergegeben ist.

### 4. Expression des ilvD-Gens

Das Plasmid pRV wurde mit den Restriktionsenzymen ScaI und XhoI, entsprechend den Angaben des Herstellers der Restriktionsenzyme, verdaut (Roche, Boehringer Mannheim). Anschließend wurde das 2,9 kb ilvD Fragment mittels Ionenaustauschersäulchen isoliert (Quiagen, Hilden). Das überhängende Ende des Xhol Schnitts des isolierten Fragmentes wurde mit Klenow Polymerase aufgefüllt. Der Vektor pJC1 (Cremer et al., Mol. Gen. Genet (1990)220:478-480) wurde PstI-geschnitten, ebenfalls mit Klenow Polymerase behandelt, und anschließend Fragment und Vektor ligiert. Mit dem Ligationsansatz wurde der E. coli Stamm DH5αmcr (Grant et al., Proceedings of the National of Sciences of the United States of America USA, 87 (1990) 4645-4649) transformiert (Hanahan, Journal of Molecular Biology 166 (1983) 557-580). Durch Plasmidpräparationen (Sambrook et al., Molecular cloning. A laboratory manual (1989) Cold Spring Harbour Laboratory Press) von Klonen wurde ein Klon identifiziert, der das rekombinante Plasmid pJC1ilvD enthielt. Mit diesem Plasmid wurde Corvnebacterium glutamicum ATCC13032 mittels Elektroporation transformiert, wie bei Haynes et al. (1989, FEMS Microbiol. Lett. 61: 329-334) beschrieben. Von Corynebacterium glutamicum ATCC13032 pJC1 und Corynebacterium glutamicum ATCC13032 pJClilvD wurde anschließend die durch ilvD kodierte Dihydroxysäuredehydrataseaktivität bestimmt. Dazu wurden die Klone in 60 ml LB-Medium kultiviert und in der exponentiellen Wachstumsphase abzentrifugiert. Das Zellpellet wurde einmal mit 0,05 M Kaliumphosphatpuffer gewaschen und im selben Puffer resuspensiert. Der Zellaufschluß erfolgte mittels 10 minütiger Ultraschallbehandlung (Branson-Sonifier W-250, Branson Sonic Power Co, Danbury, USA). Anschließend wurden die Zelltrümmer durch eine 30 minütige Zentrifugation bei 13000 rpm und 4 °C abgetrennt und der Überstand als Rohextrakt in den Enzymtest eingesetzt. Der Reaktionsansatz des Enzymtests enthielt 0,2 ml 0,25 M Tris/HCl, pH 8, 0,05 ml Rohextrakt, und 0,15 ml 65 mM alpha,β-Dihydroxy-β-methylvalerat. Die Testansätze wurden bei 30 °C inkubiert, nach 10, 20 und 30 Minuten wurde je 200 µl Proben genommen und deren Ketomethylvaleratkonzentration mittels HPLC-Analytik bestimmt (Hara et al. 1985, Analytica Chimica Acta 172: 167-173). Wie Tabelle 2 zeigt, weist der Stamm Corynebacterium glutamicum ATCC13032 pJC1ilvD eine gesteigerte Dihydroxysäuredehydrataseaktivität gegenüber dem Kontrollstamm auf.

**Tabelle 2**

| Spezifische Aktivität (µmol/min und mg Protein) der Dihydroxysäuredehydratase in Corynebacterium glutamicum ATCC13032 | |
|---|---|
| Plasmid | Dihydroxysäure dehydratase |
| pJC1 | 0,008 |
| pJC1ilvD | 0,050 |

### Beispiel 2: Konstruktion einer ilvA Deletionsmutante von Corynebacterium glutamicum

Die interne Deletion des ilvA-Gens von Corynebacterium glutamicum ATCC13032 wurde mit dem bei Schäfer et al. (Gene 145: 69-73 (1994)) beschriebenen System zum Genaustausch durchgeführt. Zur Konstruktion des Inaktivierungsvektors pK19mobsacBΔilvA wurde zunächst aus dem auf einem EcoRI-Fragment im Vektor pBM21 (Möckel et al. 1994, Molecular Microbiology 13: 833-842) vorliegenden ilvA-Gen ein internes 241 bp BglII-Fragment entfernt. Hierzu wurde der Vektor mit BglII geschnitten und, nach Abtrennung des ilvA internen BglII-Fragmentes mittels Agarosegelelektrophorese, religiert. Anschließend wurde aus dem Vektor das unvollständige Gen als EcoRI-Fragment isoliert und in den mit EcoRI linearisierten Vektor pK19mobsacB (Schäfer 1994, Gene 145: 69-73) ligiert. Der erhaltene Inaktivierungsvektor pK19mobsacBΔilvA wurde durch Transformation in den E. coli Stamm S 17-1 eingebracht (Hanahan 1983, Journal of Molecular Biology 166: 557-580) und per Konjugation nach Corynebacterium glutamicum ATCC13032 transferiert (Schäfer et al. 1990, Journal of Bacteriology 172: 1663-1666). Es wurden Kanamycin-resistente Klone von Corynebacterium glutamicum erhalten, bei denen der Inaktivierungsvektor im Genom integriert vorlag. Um auf die Excision des Vektors zu selektionieren, wurden Kanamycin-resistente Klone auf Saccharose-haltigem LB-Medium (Sambrook et al., Molecular cloning. A laboratory manual (1989) Cold Spring Harbour Laboratory Press) mit 15 g/l Agar, 2% Glucose/ 10% Saccharose ausplattiert und Kolonien erhalten, welche den Vektor durch ein zweites Rekombinationsereignis wieder verloren haben (Jäger et al. 1992, Journal of Bacteriology 174: 5462-5465). Durch Überimpfen auf Minimalmediumplatten (Medium CGXII mit 15 g/l Agar (Keilhauer et al., Journal of Bacteriology 175 (1993) 5595-5603) mit und ohne 2 mM L-Isoleucin bzw. mit und ohne 50 µg/ml Kanamycin wurden 36 Klone isoliert, welche durch die Excision des Vektors Kanamycin sensitiv und Isoleucin auxotroph waren und bei denen nun das unvollständige ilvA Gen (ΔilvA-Allel) im Genom vorlag. Ein Stamm wurde als ATCC13032ΔilvA bezeichnet und weiter verwendet.

### Beispiel 3: Klonierung der Gene der Pantothenatbiosynthese panB und panC aus C. glutamicum

### Klonierung des Operons

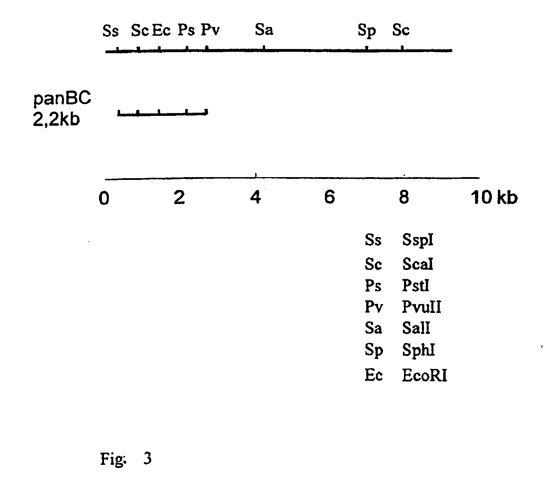

Chromosomale DNA von C. glutamicum ATCC13032 wurde isoliert und mit der Restriktionsendonuklease Sau3A geschnitten. Nach gelektrophoretischer Auftrennung wurden DNA-Fragmente in einem Größenbereich von 3 bis 7 bzw. von 9 bis 20 kb extrahiert und nachfolgend in die singuläre BamHI Schnittstelle des Vektors pBR322 ligiert. Inserttragende Kolonien wurden anhand ihrer Tetracyclinsensitivität nach Überimpfen auf LB-Platten mit 10 µg/ml Tetracyclin isoliert. Durch Plasmidpräparationen (Sambrook et al., Molecular cloning. A laboratory manual (1989) Cold Spring Harbour Laboratory Press) von gepoolten Klonen wurden 8 Plasmidpools, welche je 400 Plasmide mit einer Insertgröße von 9 bis 20 kb und 9 Plasmidpools, welche je 500 Plasmide mit einer Insertgröße von 3 bis 7 kb enthielten, isoliert. Die E. coli panB Mutante SJ2 (Cronan et al. 1982, J. Bacteriol. 149: 916-922) wurde mit dieser Genbank mittels Elektroporation (Wehrmann et al. 1994, Microbiology 140: 3349-3356) transformiert. Die Transformationsansätze wurden direkt auf CGXII-Medium (J. Bacteriol. (1993) 175: 5595-5603) ausplattiert. Von Klonen, welche in der Lage waren ohne Pantothenatsupplementation zu wachsen, wurde Plasmid-DNA isoliert (Sambrook et al. 1989) und durch Retransformation wurden 8 Klone erhalten, deren D-Pantothenatbedürftigkeit bestätigt wurde. Mit den 8 Plasmiden wurde eine Restriktionskartierung durchgeführt. Einer der untersuchten Vektoren, im Folgendem pUR1 genannt enthielt ein Insert von 9,3 kb (Figur 3). Die Transformation der E. coli panC_Mutante DV39 (Vallari et al. 1985, J. Bacteriol. 164: 136-142) ergab, daß der Vektor pUR1 ebenfalls in der Lage war, den panC Defekt dieser Mutante zu komplementieren. Ein 2,2 kb großes Fragment des Inserts von pUR1 wurde nach der Dideoxy-Kettenabbruchmethode von Sanger et al. sequenziert (Proc. Natl. Acad. Sci. USA (1977) 74: 5463-5467). Die gelelektrophoretische Analyse erfolgte mit dem automatischem Laser-Fluoreszenz Sequenziergerät (A.L.F.) von Amersham Pharmacia Biotech (Uppsala, Schweden). Die erhaltene Nukleotidsequenz wurde mit dem Programmpaket HUSAR (Release 4.0, EMBL, Cambridge, GB) analysiert. Die Nukleotidsequenz ist als SEQ ID No. 3 wiedergegeben. Die Analyse ergab die Identizierung von zwei offenen Leserastern. Ein offenes Leseraster umfaßt 813 Basenpaare und weist hohe Homologien zu bereits bekannten panB-Genen aus anderen Organismen aufweist. Das panB-Gen aus C. glutamicum kodiert für ein Polypeptid von 271 Aminosäuren (siehe SEQ ID No. 4). Das zweite offene Leseraster umfaßt 837 Basenpaare und weist hohe Homologien zu bereits bekannten panC-Genen aus anderen Organismen aufweist. Das panC-Gen aus C. glutamicum kodiert für ein Polypeptid von 279 Aminosäuren (siehe SEQ ID No. 5).

### Beispiel 4: Konstruktion einer panBC Deletionsmutante von Corynebacterium glutamicum

Das genomische panBC-Fragment von Corynebacterium glutamicum ATCC13032 sowie Corynebacterium glutamicum ATCC13032ΔilvA wurde mit dem bei Schäfer et al. (Gene 145: 69-73 (1994)) beschriebenen System zum Genaustausch durchgeführt. Zur Konstruktion des Deletionsvektors pK19mobsacBΔpanBC wurde zunächst das 3.95 kb große SspI/SaII Fragment mit panBC mit pUC18 ligiert, der zuvor SmaI/SalI geschnitten worden war. Anschließend wurde ein 1293 bp großes EcoRV/NruI Fragment aus dem überlappenden Bereich der panBC Gene durch Restriktionsverdau und Religation entfernt. Um die Umklonierung in pK19mobsacB zu ermöglichen, wurde mit den 2 Primern 5'-GAGAACTTAATCGAGCAACACCCCTG, 5'-GCGCCACGCCTAGCCTTGGCCCTCAA und der Polymerasekettenreaktion (PCR) der deletierte panBC-Bereich in pUC18 amplifiziert, um so ein 0,5 kb ΔpanBC Fragment zu erhalten, das an den Enden eine SaII, bezw. EcoRI Schnittstelle trägt. Die PCR wurde nach Sambrook et al. (Molecular cloning. A laboratory manual (1989) Cold Spring Harbour Laboratory Press) mit einer Annealingtemperatur von 55 °C durchgeführt. Das erhaltene Fragment wurde mit dem Vektor pK19mobsac ligiert, der zuvor EcoRI/SalI geschnitten und mit alkalischer Phosphatase behandelt worden war. Der erhaltene Inaktivierungsvektor pK19mobsacBΔpanBC wurde durch Transformation in den Escherichia coli Stamm S 17-1 eingebracht (Hanahan (1983) J. Mol. Biol. 166: 557-580) und per Konjugation nach Corynebacterium glutamicum ATCC13032 transferiert (Schäfer et al. (1990) J. Bacteriol. 172: 1663-1666). Es wurden Kanamycin-resistente Klone von Corynebacterium glutamicum erhalten, bei denen der Inaktivierungsvektor im Genom integriert vorlag. Um auf die Excision des Vektors zu selektionieren, wurden Kanamycin-resistente Klone auf Saccharose-haltigem LB-Medium (Sambrook et al., Molecular cloning. A laboratory manual (1989) Cold Spring Harbour Laboratory Press) mit 15 g/l Agar, 2% Glucose/ 10% Saccharose ausplattiert und Kolonien erhalten, welche den Vektor durch ein zweites Rekombinationsereignis wieder verloren haben (Jäger et al. 1992, Journal of Bacteriology 174: 5462-5465). Durch Überimpfen auf Minimalmediumplatten (Medium CGXII mit 15 g/l Agar (Keilhauer et al., Journal of Bacteriology 175 (1993) 5595-5603) mit und ohne 2 mM L-Isoleucin bzw. mit und ohne 50 Hg/ml Kanamycin wurden 36 Klone isoliert, welche durch die Excision des Vektors Kanamycin sensitiv und Isoleucin auxotroph waren und bei denen nun die Sequenz der unvollständigen pan Gene (ΔpanBC-Allele) im Genom vorlag. Ein Stamm wurde als ATCC13032ΔpanBC bezeichnet. Auf die gleiche Weise wie in diesem Beispiel detailliert beschrieben wurde auch die panBC Deletion in ATCC13032ΔilvA eingeführt, um den Stamm ATCC13032ΔilvAΔpanBC zu erhalten.

### Beispiel 5: Expression der Gene ilvD, ilvBN, und ilvC in Corynèbacterium glutamicum

Die Gene der Acetohydroxysäuresynthase (ilvBN) und der Isomeroreduktase (ilvC) (Cordes et al. 1992, Gene 112: 113-116 und Keilhauer et al. 1993, Journal of Bacteriology 175: 5595-5603) und der Dihydroxysäuredehydratase (ilvD) (Beispiel 1) wurden zur Expression in den Vektor pECM3 kloniert. Der Vektor pECM3 ist ein Derivat von pECM2 (Jäger et al. 1992, Journal of Bacteriology 174: 5462-5465), das durch Deletion des ca. 1 kbp langen BamHI/BglII DNA-Fragmentes entstand, welches das Kanamycinresistenzgen trägt.

In dem Vektor pKK5 (Cordes et al. 1992, Gene 112:113-116) lagen die Gene ilvBNC bereits im Vektor pJC1 (Cremer et al. 1990, Molecular and General Genetics 220: 478-480) kloniert vor. Aus diesem wurde ein 5,7 kb XbaIilvBNC-Fragment isoliert und zusammen mit einem, das ilvD-Gen enthaltende, 3,1 kb-XbaI Fragment des Vektors pRV in den mit XbaI linearisierten Vektor pECM3 eingebracht. Der Ligationsansatz wurde hierbei in den E. coli Stamm DH5αmcr transformiert. Aus einem Klon wurde das Plasmid pECM3ilvBNCD erhalten.

Mittels Elektroporation (Haynes 1989, FEMS Microbiology Letters 61: 329-334) und Selektion auf Chloramphenicolresistenz (3 µg/ml) wurde das Plasmid pECM3ilvBNCD in den Stamm ATCC13032ΔilvA eingebracht und der Stamm ATCC13032ΔilvA/pECM3ilvBNCD erhalten.

### Beispiel 6: Produktion von L-VaIin mit verschiedenen Corynebacterium glutamicum Stämmen

Zur Untersuchung ihrer Valinbildung wurden die in Tabelle 4 angegebenen Stämme in 60 ml Brain Heart Infusion-Medium (Difco Laboratories, Detroit, USA) für 14 h bei 30 °C vorkultiviert. Anschließend wurden die Zellen einmal mit 0,9% NaCl-Lösung (w/v) gewaschen und mit dieser Suspension je 60 ml CgXII-Medium so angeimpft, daß die OD600 0,5 betrug. Das Medium war identisch mit dem bei Keilhauer et al., (Journal of Bacteriology (1993) 175: 5595-5603) beschriebenen Medium. Für die Kultivierung der ΔilvA Stämme enthielt das Medium aber zusätzlich 250 mg/l L-Isoleucin. Es ist in Tabelle 3 dargestellt.

**Tabelle 3**

| Zusammensetzung des Mediums CGXII | |
|---|---|
| Komponente | Konzentration |
| (NH₄)₂SO₄ | 20 g/L |
| Harnstoff | 5 g/L |
| KH₂PO₄ | 1 g/L |
| K₂HPO₄ | 1 g/L |
| Mg₂O₄*7 H₂O | 0,25 g/L |
| 3-Morpholinopropansulfonsäure | 42 g/L |
| CaCl₂ | 10 mg/L |
| FeSO₄*7 H₂O | 10 mg/L |
| MnSO_{4*} H₂O | 10 mg/L |
| ZnSO_{4*}7 H₂O | 1 mg/L |
| CuSO₄ | 0,2 mg/L |
| NiCl₂*6 H₂O | 0,02 mg/L |
| Biotin (pH7) | 0,2 mg/L |
| Glukose | 40 g/L |
| Protokatechusäure | 0,03 mg/L |

Nach 48 stündiger Kultivierung wurden Proben genommen, die Zellen abzentrifugiert und der Überstand sterilfiltriert. Die L-Valinkonzentration des Überstands wurde mit Hilfe der Hochdruckflüssigchromatografie mit integrierter Vorsäulenderivatisierung der Aminosäure mit o-Phthdialdehyd wie bei Jones und Gilligan (J. Chromatogr. 266 (1983) 471-482) bestimmt. Die Ergebnisse sind in Tabelle 4 dargestellt.

**Tabelle 4**

| L-Valinproduktion mit verschiedenen Corynebacterium glutamicum Stämmen | |
|---|---|
| C. glutamicum | L-Valin (mM) |
| ATCC 13032 | 0,5 |
| ATCC 13032 pJClilvD | 2,2 |
| ATCC 13032 pJC1ilvBNC | 20,0 |
| ATCC 13032 pJClilvBNCD | 26,2 |
| ATCC 13032 ΔilvA | 2,7 |
| ATCC 13032 ΔilvA pJClilvD | 7,0 |
| ATCC 13032 ΔilvA pJC1ilvBNCD | 28,5 |
| ATCC 13032 ΔpanBC | 8,2 |
| ATCC 13032 ΔilvAΔpanBC | 31,1 |
| ATCC 13032 ΔilvAΔpanBC pJC1ilvBNCD | 72,7 |

## Patentansprüche

1. Verfahren zur mikrobiellen Herstellung von L-Valin, bei dem die Dihydroxysäuredehydratase- (ilvD) Aktivität und/oder die ilvD-Genexpression in einem Mikroorganismus verstärkt wird,
**dadurch gekennzeichnet,**
**dass** die Aktivität eines oder mehrerer, an der Synthese von D-Pantothenat spezifisch beteiligter, Enzyme abgeschwächt oder ausgeschaltet ist.

2. Verfahren nach Anspruch 1, bei dem zusätzlich die Acetohydroxysäuresynthase- (ilvRN) und Isomeroreduktase- (ilvC) Aktivität und/oder ilvBNC-Genexpression im Mikroorganismus verstärkt wird.

3. Verfahren nach Anspruch 1 oder 2,
**dadurch gekennzeichnet,**
**dass** die endogene ilvD- oder ilvBNCD-Aktivität im Mikroorganismus erhöht wird.

4. Verfahren nach Anspruch 3,
**dadurch gekennzeichnet,**
**dass** durch Mutation des endogenen ilvD-Gens oder der ilvBNCD-Gene entsprechende Enzyme mit höherer Aktivität erzeugt werden.

5. Verfahren nach einem oder mehreren der vorhergehenden Ansprüche,
**dadurch gekennzeichnet,**
**dass** die ilvD- oder ilvBNDC-Genexpression durch Erhöhen der Genkopienzahl verstärkt wird.

6. Verfahren nach Anspruch 5,
**dadurch gekennzeichnet,**
**dass** zur Erhöhung der Genkopienzahl das ilvD-Gen oder die ilvBNCD-Gene in ein Genkonstrukt eingebaut werden.

7. Verfahren nach Anspruch 6,
**dadurch gekennzeichnet,**
**dass** ein Mikroorganismus mit dem das ilvD-Gen oder die ilvBNCD-Gene enthaltende Genkonstrukt transformiert wird.

8. Verfahren nach Anspruch 7,
**dadurch gekennzeichnet,**
**dass** als Mikroorganismus Corynebacterium glutamicum verwendet wird.

9. Verfahren nach einem oder mehreren der vorhergehenden Ansprüche,
**dadurch gekennzeichnet,**
**dass** ein Mikroorganismus verwendet wird, in dem die Aktivität zumindest eines Enzyms, das an einem Stoffwechselweg beteiligt ist, der die L-ValinBildung herabsetzt, abgeschwächt oder ausgeschaltet ist.

10. Verfahren nach Anspruch 9,
**dadurch gekennzeichnet,**
**dass** die Aktivität des an der Synthese von L-Isoleucin beteiligten Enzyms Threonindehydratase (ilvA) abgeschwächt oder ausgeschaltet ist.

11. Verfahren nach einem der Ansprüche 1-10,
**dadurch gekennzeichnet,**
**dass** die Aktivität des Enzyms Ketopanthoathydroxymethyltransferase (pan B) und/oder des Enzyms Panthothenatligase (pan C) abgeschwächt oder ausgeschaltet ist.

12. Mit einem Genkonstrukt, enthaltend das ilvD-Gen oder die ilvBNCD-Gene, transformierter Mikroorganismus, in dem die Aktivität eines oder mehrerer, an der Synthese von D-Pantothenat spezifisch beteiligter, Enzyme abgeschwächt oder ausgeschaltet ist.

13. Transformierter Mikroorganismus nach Anspruch 12, in dem die Aktivität des Enzyms Ketopanthoathydroxymethyltransferase (pan B) und/oder des Enzyms Panthothenatligase (pan C) abgeschwächt oder ausgeschaltet ist.

14. Transformierter Mikroorganismus nach Anspruch 12 oder 13, in dem die Aktivität des an der Synthese von L-Isoleucin beteiligten Enzyms Threonindehydratase (ilvA) abgeschwächt oder ausgeschaltet ist.

15. Transformierter Mikroorganismus nach einem oder mehreren der Ansprüche 12 bis 14,
**gekennzeichnet durch** Corynebacterium glutamicum.

## Claims

1. Process for the microbial production of L-valine, in which the dihydroxy acid dehydratase (ilvD) activity and/or the ilvD gene expression in a microorganism is intensified, **characterised in that** the activity of one or more enzymes specifically involved in the synthesis of D-pantothenate is attenuated or turned off.

2. Process according to claim 1, wherein in addition the acetohydroxy acid synthase (ilvBN) activity and isomeroreductase (ilvC) activity and/or ilvBNC gene expression in the microorganism is intensified.

3. Process according to claim 1 or 2, **characterised in that** the endogenous ilvD or ilvBNCD activity in the microorganism is increased.

4. Process according to claim 3, **characterised in that** by mutation of the endogenous ilvD gene or ilvBNCD genes corresponding enzymes with higher activity are created.

5. Process according to one or more of the foregoing claims, **characterised in that** the ilvD or ilvBNCD gene expression is intensified by increasing the gene copy number.

6. Process according to claim 5, **characterised in that**, to increase the gene copy number, the ilvD gene or the ilvBNCD genes is/are incorporated into a gene construct.

7. Process according to claim 6, **characterised in that** a microorganism is transformed with the gene construct containing the ilvD gene or ilvBNCD genes.

8. Process according to claim 7, **characterised in that** Corynebacterium glutamicum is used as the microorganism.

9. Process according to one or more of the foregoing claims, **characterised in that** a microorganism is used in which the activity of at least one enzyme which is involved in a metabolic path which decreases L-valine formation is attenuated or turned off.

10. Process according to claim 9, **characterised in that** the activity of the enzyme threonine dehydratase (ilvA) involved in the synthesis of L-isoleucine is attenuated or turned off.

11. Process according to one of claims 1 - 10, **characterised in that** the activity of the enzyme ketopantoate hydroxymethyl transferase (pan B) and/or the enzyme pantothenate ligase (pan C) is attenuated or turned off.

12. Microorganism transformed with a gene construct containing the ilvD gene or the ilvBNCD genes, wherein the activity of one or more enzymes specifically involved in the synthesis of D-pantothenate is attenuated or turned off.

13. Transformed microorganism according to claim 12, wherein the activity of the enzyme ketopantoate hydroxymethyl transferase (pan B) and/or the enzyme pantothenate ligase (pan C) is attenuated or turned off.

14. Transformed microorganism according to claim 12 or 13, wherein the activity of the enzyme threonine dehydratase (ilvA) involved in the synthesis of L-isoleucine is attenuated or turned off.

15. Transformed microorganism according to one or more of claims 12 to 14, **characterised by** Corynebacterium glutamicum.

## Revendications

1. Procédé pour la production microbienne de L-valine, dans lequel l'activité de dihydroxy-acide-déhydratase(ilvD) et/ou l'expression génétique de ilvD est amplifiée dans un microorganisme
**caractérisé en ce que**
l'activité d'une ou de plusieurs enzyme(s) participant de manière spécifique à la synthèse de la D-pantothénate est atténué ou bloquée.

2. Procédé selon la revendication 1, dans lequel en outre l'activité d'acétohydroxy-acide synthase(ilvBN) et d'isomèro-réductase(ilVC) et/ou l'expression génétique de ilvNBC est amplifiée dans un microorganisme.

3. Procédé selon la revendication 1 ou 2,
**caractérisé en ce que**
l'activité endogène de ilvD ou de ilvBNCD est accrue dans le microorganisme.

4. Procédé selon la revendication 3,
**caractérisé en ce que,**
par mutation du gène ilvD endogène ou des gènes ilvBNCD, des enzymes correspondantes sont produites avec une activité accrue.

5. Procédé selon l'une ou plusieurs des revendications précédentes,
**caractérisé en ce que**
l'expression génétique de ilvD ou de ilvBNCD est amplifiée par l'augmentation du nombre de copies du gène.

6. Procédé selon la revendication 5,
**caractérisé en ce que,**
pour augmenter le nombre de copie du gène, le gène ilvD ou les gènes ilvBNCD sont insérés dans une construction génétique.

7. Procédé selon la revendication 6,
**caractérisé en ce qu'**
un microorganisme est transformé avec la construction génétique comprenant le gène ilv-D ou les gènes ilvBNCD.

8. Procédé selon la revendication 7,
**caractérisé en ce que**
*Corynebacterium glutamicum* est utilisé en tant que microorganisme.

9. Procédé selon l'une ou plusieurs des revendications précédentes,
**caractérisé en ce qu'**
un microorganisme est utilisé, dans lequel l'activité d'au moins une enzyme qui participe à une voie métabolique réduisant la formation de L-Valine est atténuée ou bloquée.

10. Procédé selon la revendication 9,
**caractérisé en ce que**
l'activité de l'enzyme thréonindéhydratase (ilvA) participant à la synthèse de la L-isoleucine est atténuée ou bloquée.

11. Procédé selon l'une des revendications 1 à 10,
**caractérisé en ce que**
l'activité de l'enzyme kétopanto-athydroxyméthyl-transférase (pan B) et/ou de l'enzyme pantothénate-ligase (pan C) est atténuée ou bloquée.

12. Microorganisme transformé, avec une construction génétique, contenant le gène ilvD ou les gènes ilvBNCD, dans lequel l'activité d'une ou de plusieurs enzyme(s) participant de manière spécifique à la synthèse du D-pantothénate est atténuée ou bloquée.

13. Microorganisme transformé selon la revendication 12, dans lequel l'activité de l'enzyme kétopanto-athydroxyméthyl-transférase (pan B) et/ou de l'enzyme pantothénate-ligase (pan C) est atténuée ou bloquée.

14. Microorganisme transformé selon la revendication 12 ou 13, dans lequel l'activité de l'enzyme thréonine-déhydratase (ilvA) participant à la synthèse de L-isoleucine est atténuée ou bloquée.

15. Microorganisme transformé selon l'une ou plusieurs des revendications 12 à 14, **caractérisé en ce qu'**il s'agit de *Corynebacterium glutamicum*.


### SEQUENZPROTOKOLL

(1) ALLGEMEINE ANGABEN:
   (i) ANMELDER:
      (A) NAME: Forschungszentrum Juelich GmbH
      (B) STRASSE: Postfach 1913
      (C) ORT: Juelich
      (E) LAND: Deutschland
      (F) POSTLEITZAHL: 52425
      (G) TELEFON: 02461/614480
      (H) TELEFAX: 02461/612860
   (ii) BEZEICHNUNG DER ERFINDUNG: Valinherstellung
   (iii) ANZAHL DER SEQUENZEN: 5
   (iv) COMPUTER-LESBARE FASSUNG:
      (A) DATENTRéGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LéNGE: 2952 Basenpaare
      (B) ART: Nucleotid
      (C) STRANGFORM: Einzelstrang
      (D) TOPOLOGIE: linear
   (ii) ART DES MOLEKöLS: Genom-DNA
   (iii) HYPOTHETISCH: NEIN
   (iv) ANTISENSE: NEIN
   (xi) SEQUENZBESCHREIBUNG: SEQ ID NO: 1: 
AGTACTTGGA GCGCCAAAAG GCACTGGGCA AGCCAGTTCA GTTGAACTTC GATGACGACA
CCGATGGGAA TACAACACAA ACAGAAAGCG TTGAATCCCA AGAGACCGGA CAAGCCGCGT
CTGAAACCTC ACATCGTGAT AACCCTGCGT CACAGCACTA GAGTGTAATA AGCCGTCCGA
ACCAAAGGTC CACACCTCTG CACGAGTAGA AGCTCACCCA AGTTTTCAAA GTGCCGTTGA
 
TTCTTGACAA CCACCCGCCG CTCTTTAGAG CAGATTTGAA AAGCGCATCA TGATCCCACT
TCGTTCAAAA GTCACCACCG TCGGTCGCAA TGCAGCTGGC GCTCGCGCCC TTTGGCGTGC
CACCGGCACC AAGGAAAATG AGTTCGGCAA GCCAATTGTT GCCATCGTAA ACTCCTACAC
CCAGTTCGTG CCCGGACACG TTCACCTTAA GAACGTCGGC GATATTGTGG CAGATGCAGT
GCGCAAAGCC GGTGGCGTTC CAAAGGAATT CAACACCATC GTCGATGACG GCATCGCCAT
GGGACACGGC GGCATGCTGT ACTCCCTGCC ATCCCGTGAA ATCATCGCCG ACTCCGTCGA
ATACATGGTC AACGCACACA CCGCCGACGC CATGGTGTGT ATCTCCAACT GTGACAAGAT
CACCCCAGGC ATGCTCAACG CAGCAATGCG CCTGAACATC CCAGTGGTCT TCGTTTCCGG
TGGCCCAATG GAAGCTGGCA AGGCTGTCGT CGTTGAGCGC GTTGCACACG CACCAACCGA
CCTCATCACC GCGATCTCCG CATCCGCAAG CGATGCAGTC GACGACGCAG GCCTTGCAGC
CGTTGAACGA TCCGCATGCC CAACCTGTGG CTCCTGCTCC GGTATGTTCA CCGCGAACTC
CATGAACTGC CTCACCGAAG CTCTGGGACT TTCTCTCCCG GGCAACGGCT CCACTCTGGC
AACCCACGCA GCACGTCGCG CACTGTTTGA AAAGGCCGGC GAAACCGTCG TTGAACTGTG
CCGCCGCTAC TACGGTGAAG AAGACGAATC CGTTCTGCCA CGTGGCATTG CCACCAAGAA
GGCATTCGAA AACGCAATGG CACTGGATAT GGCCATGGGT GGATCCACCA ACACCATCCT
CCACATCCTC GCAGCTGCCC AGGAAGGCGA AGTTGACTTC GACCTCGCAG ACATCGACGA
ACTGTCCAAA AACGTCCCCT GCCTGTCCAA GGTTGCACCA AACTCCGACT ACCACATGGA
AGACGTCCAC CGCGCCGGTC GCATTCCAGC ACTGCTCGGC GAGCTCAACC GCGGTGGCCT
GCTGAACAAG GACGTCCACT CCGTTCACTC CAACGACCTT GAAGGTTGGT TGGATGACTG
GGATATCCGC TCTGGCAAGA CCACCGAAGT AGCAACCGAA CTCTTCCACG CAGCCCCAGG
TGGCATCCGC ACCACCGAAG CATTCTCCAC CGAGAACCGC TGGGACGAAC TCGACACCGA
CGCTGCCAAG GGCTGCATCC GCGACGTTGA ACACGCCTAC ACCGCCGACG GCGGCCTGGT
TGTTCTTCGC GGCAACATCT CCCCTGACGG CGCAGTGATC AAGTCCGCAG GTATCGAAGA
AGAGCTGTGG AACTTCACCG GACCAGCACG AGTTGTCGAA AGCCAGGAAG AGGCAGTCTC
TGTCATCCTG ACCAAGACCA TCCAAGCTGG CGAAGTTCTG GTCGTCCGCT ACGAAGGCCC
ATCAGGTGGA CCAGGCATGC AGGAAATGCT TCACCCAACC GCATTCCTCA AGGGATCCGG
CCTGGGCAAG AAGTGTGCAC TGATCACCGA CGGCCGTTTC TCCGGAGGTT CCTCAGGACT
 
GTCCATCGGC CACGTCTCCC CAGAAGCAGC ACACGGCGGA GTCATTGGTC TGATCGAAAA
CGGCGACATC GTCTCCATCG ACGTTCACAA CCGCAAGCTC GAAGTTCAGG TCTCCGACGA
GGAACTCCAG CGCCGCCGCG ACGCTATGAA CGCCTCCGAG AAGCCATGGC AGCCAGTCAA
CCGTAACCGC GTTGTCACCA AGGCACTGCG CGCATACGCA AAGATGGCTA CCTCCGCTGA
TAAGGGTGCA GTCCGTCAGG TCGACTAACC CTTTGTGAGT GTTTGAGCAC CGGTTCCCTA
CTTTGGGTTC CGGTGCTTTT TCATGTCTTG GCCTGTGTGG GCGTGGTGGA GCTCCCCGTT
GCAAATACTC ACCACAAGTT GCAGGATTTC TGCTGGTTGT GGTGGATTTT CCCGCTTTAT
AGCCCTATGC GTGCAACTTT CGGACCGATT CCAAAGGGCA AAGCCCTGTT TGTGGTGGAT
CCTTGCCCTG GAAGCTTTCA GGAACCACAA CTACCCCACT GACCCCAAAG TGGATAGGCC
CTATTCTTCC GTTTAAGCGC CTCAAACACC TCTCCCCACA CTTGACCCAT TAGGCAATTA
CGAATCCTTA AACAGCCTTC TACAGCACCA TGCCCCAAAC CGAACCCAGG CATGAAAAAG
ACCCTCACCA GGAGGGTCTT TTTCTAAAAC TTTGGCTACG CGATTGGGTT CACACCCGCA
CCGAACCACC ACAGCAGAAC TGCCGCTGCG ATGCCGATGA CCACGAAGAT CCACGAGCTC
ACCAGTGGAC GCTTTGCCCA ACCTCGGCCA GAGTCAAGGG AAATCTTGCC GGGGCCGGTG
2700 AACTGAAGTC CGACAACCAC GATAGTGAGG ATCAGTGCCA GCATCAATGG CTCACTAAGT
2760 TCACCCCAAC CACCTTCATG AGTGTTGACT TGGTGAAGGG TGGTAAAGGA TGTCGCCACC
2820 GTGGCTACCG CTGCTGCCAC TGGGGTCATC AGACCAAGGA GCAGGAAGAC ACCAGCCGCA
2880 AGTTCAATAG ATGGAAGCAG GATCGCGAGG ATTTCAGGCC ACTGGTAACC AGCGAACTCT
2940 GCCTCGACTC TA
2952
(2) ANGABEN ZU SEQ ID NO: 2:
   (i) SEQUENZKENNZEICHEN:
      (A) LéNGE: 612 AminosÑuren
      (B) ART: AminosÑure
      (C) STRANGFORM: Einzelstrang
      (D) TOPOLOGIE: linear
   (ii) ART DES MOLEKöLS: Protein
   (iii) HYPOTHETISCH: NEIN
   (iv) ANTISENSE: NEIN
   (xi) SEQUENZBESCHREIBUNG: SEQ ID NO: 2: 
Met Ile Pro Leu Arg Ser Lys Val Thr Thr Val Gly Arg Asn Ala
Ala 1 5 10
15

Gly Ala Arg Ala Leu Trp Arg Ala Thr Gly Thr Lys Glu Asn Glu
Phe 20 25 30

Gly Lys Pro Ile Val Ala Ile Val Asn Ser Tyr Thr Gln Phe Val
Pro 35 40 45

Gly His Val His Leu Lys Asn Val Gly Asp Ile Val Ala Asp Ala
Val 50 55 60

Arg Lys Ala Gly Gly Val Pro Lys Glu Phe Asn Thr Ile Val Asp
Asp 65 70 75
80

Gly Ile Ala Met Gly His Gly Gly Met Leu Tyr Ser Leu Pro Ser
Arg 85 90
95

Glu Ile Ile Ala Asp Ser Val Glu Tyr Met Val Asn Ala His Thr
Ala 100 105 110

Asp Ala Met Val Cys Ile Ser Asn Cys Asp Lys Ile Thr Pro Gly
Met 115 120 125

Leu Asn Ala Ala Met Arg Leu Asn Ile Pro Val Val Phe Val Ser
Gly 130 135 140

Gly Pro Met Glu Ala Gly Lys Ala Val Val Val Glu Arg Val Ala
His 145 150 155
160

Ala Pro Thr Asp Leu Ile Thr Ala Ile Ser Ala Ser Ala Ser Asp
Ala 165 170
175

Val Asp Asp Ala Gly Leu Ala Ala Val Glu Arg Ser Ala Cys Pro
Thr 180 185 190

Cys Gly Ser Cys Ser Gly Met Phe Thr Ala Asn Ser Met Asn Cys
Leu 195 200 205

Thr Glu Ala Leu Gly Leu Ser Leu Pro Gly Asn Gly Ser Thr Leu
Ala 210 215 220

Thr His Ala Ala Arg Arg Ala Leu Phe Glu Lys Ala Gly Glu Thr
Val 225 230 235
240

Val Glu Leu Cys Arg Arg Tyr Tyr Gly Glu Glu Asp Glu Ser Val
 
Leu 245                    250
255

Pro Arg Gly Ile Ala Thr Lys Lys Ala Phe Glu Asn Ala Met Ala
Leu 260                    265                    270

Asp Met Ala Met Gly Gly Ser Thr Asn Thr Ile Leu His Ile Leu
Ala 275                    280                    285

Ala Ala Gln Glu Gly Glu Val Asp Phe Asp Leu Ala Asp Ile Asp
Glu 290                    295                    300

Leu Ser Lys Asn Val Pro Cys Leu Ser Lys Val Ala Pro Asn Ser
Asp 305                    310                    315
320

Tyr His Met Glu Asp Val His Arg Ala Gly Arg Ile Pro Ala Leu
Leu 325                    330
335

Gly Glu Leu Asn Arg Gly Gly Leu Leu Asn Lys Asp Val His Ser
Val 340                    345                    350

His Ser Asn Asp Leu Glu Gly Trp Leu Asp Asp Trp Asp Ile Arg
Ser 355                    360                    365

Gly Lys Thr Thr Glu Val Ala Thr Glu Leu Phe His Ala Ala Pro
Gly 370                    375                    380

Gly Ile Arg Thr Thr Glu Ala Phe Ser Thr Glu Asn Arg Trp Asp
Glu 385                    390                    395
400

Leu Asp Thr Asp Ala Ala Lys Gly Cys Ile Arg Asp Val Glu His
Ala 405                    410
415

Tyr Thr Ala Asp Gly Gly Leu Val Val Leu Arg Gly Asn Ile Ser
Pro 420                    425                    430

Asp Gly Ala Val Ile Lys Ser Ala Gly Ile Glu Glu Glu Leu Trp
Asn 435                    440                    445

Phe Thr Gly Pro Ala Arg Val Val Glu Ser Gln Glu Glu Ala Val
Ser 450                    455                    460

Val Ile Leu Thr Lys Thr Ile Gln Ala Gly Glu Val Leu Val Val
Arg 465                    470                    475
480

Tyr Glu Gly Pro Ser Gly Gly Pro Gly Met Gln Glu Met Leu His
Pro 485                    490
495
 
Thr Ala Phe Leu Lys Gly Ser Gly Leu Gly Lys Lys Cys Ala Leu
Ile 500 505 510

Thr Asp Gly Arg Phe Ser Gly Gly Ser Ser Gly Leu Ser Ile Gly
His 515 520 525

Val Ser Pro Glu Ala Ala His Gly Gly Val Ile Gly Leu Ile Glu
Asn 530 535 540

Gly Asp Ile Val Ser Ile Asp Val His Asn Arg Lys Leu Glu Val
Gln 545 550 555
560

Val Ser Asp Glu Glu Leu Gln Arg Arg Arg Asp Ala Met Asn Ala
Ser 565 570
575

Glu Lys Pro Trp Gln Pro Val Asn Arg Asn Arg Val Val Thr Lys
Ala 580 585 590

Leu Arg Ala Tyr Ala Lys Met Ala Thr Ser Ala Asp Lys Gly Ala
Val 595 600 605

Arg Gln Val Asp
610
(2) ANGABEN ZU SEQ ID NO: 3:
   (i) SEQUENZKENNZEICHEN:
      (A) LéNGE: 2164 Basenpaare
      (B) ART: Nucleotid
      (C) STRANGFORM: Einzelstrang
      (D) TOPOLOGIE: linear
   (ii) ART DES MOLEKöLS: Genom-DNA
   (iii) HYPOTHETISCH: NEIN
   (iv) ANTISENSE: NEIN
   (xi) SEQUENZBESCHREIBUNG: SEQ ID NO: 3: 
GCTTCGGGGT ACCAATTCCT TTAAGAACCA TCAGATCAAT CTGTTGTACA TTCTCGGCCA
GATTCAGCTT TTCGGTAAGG ACGAAACACT TTCACTTGAA TCGGCAGCAA AGTTTCTTAA
AGTTTCTAAG GCAACTGCAA CGAGGTATTT TAGAACTCTC CGAGAAATGG AATTAGTTCA
CGAGGTCAGC AAACGCCCTT TGCGGTTTGC GCTCACGGAT AAAGGTCGTG AGATAGTAGG
TCTTGAGGTA AAAATTTGAC TCCATAACGA GAACTTAATC GAGCAACACC CCTGAACAGT
GAATCAAATC GGAATTTATT TATTCTGAGC TGGTCATCAC ATCTATACTC ATGCCCATGT
 
CAGGCATTGA TGCAAAGAAA ATCCGCACCC GTCATTTCCG CGAAGCTAAA GTAAACGGCC
AGAAAGTTTC GGTTCTCACC AGCTATGATG CGCTTTCGGC GCGCATTTTT GATGAGGCTG
GCGTCGATAT GCTCCTTGTT GGTGATTCCG CTGCCAACGT TGTGCTGGGT CGCGATACCA
CCTTGTCGAT CACCTTGGAT GAGATGATTG TGCTGGCCAA GGCGGTGACG ATCGCTACGA
AGCGTGCGCT TGTGGTGGTT GATCTGCCGT TTGGTACCTA TGAGGTGAGC CCAAATCAGG
CGGTGGAGTC CGCGATCCGG GTCATGCGTG AAACGGGTGC GGCTGCGGTG AAGATCGAGG
GTGGCGTGGA GATCGCGCAG ACGATTCGAC GCATTGTTGA TGCTGGAATT CCGGTTGTCG
GCCACATCGG GTACACCCCG CAGTCCGAGC ATTCCTTGGG CGGCCACGTG GTTCAGGGTC
GTGGCGCGAG TTCTGGAAAG CTCATCGCCG ATGCCCGCGC GTTGGAGCAG GCGGGTGCGT
TTGCGGTTGT GTTGGAGATG GTTCCAGCAG AGGCAGCGCG CGAGGTTACC GAGGATCTTT

CCATCACCAC TATCGGAATC GGTGCCGGCA ATGGCACAGA TGGGCAGGTT TTGGTGTGGC
AGGATGCCTT CGGCCTCAAC CGCGGCAAGA AGCCACGCTT CGTCCGCGAG TACGCCACCT
TGGGCGATTC CTTGCACGAC GCCGCGCAGG CCTACATCGC CGATATCCAC GCGGGTACCT
TCCCAGGCGA AGCGGAGTCC TTTTAATGCA GGTAGCAACC ACAAAGCAGG CGCTTATCGA
CGCCCTCCTC CACCACAAAT CCGTCGGGCT CGTCCCCACC ATGGGTGCGC TACACAGCGG
ACACGCCTCG TTGGTTAAAG CAGCACGCGC TGAAAACGAC ACTGTTGTAG CCAGTATTTT
TGTCAATCCC CTGCAGTTTG AAGCACTCGG TGATTGCGAT GATTACCGCA ACTATCCCCG
CCAACTCGAC GCCGATTTAG CACTGCTTGA AGAGGCAGGT GTGGATATTG TGTTCGCACC
CGATGTGGAG GAAATGTACC CCGGTGGCTT GCCACTAGTG TGGGCGCGCA CCGGTTCCAT
CGGAACAAAA TTGGAGGGTG CCAGCAGGCC TGGCCATTTC GATGGTGTGG CTACCGTGGT
GGCGAAGCTG TTCAATTTGG TGCGCCCTGA TCGTGCATAT TTTGGACAAA AAGATGCTCA
GCAGGTTGCG GTGATTCGGC GATTGGTTGC CGATCTAGAC ATTCCCGTGG AGATTCGTCC
CGTTCCGATT ATTCGTGGCG CCGATGGCTT AGCCGAATCC AGCCGCAATC AACGTCTTTC
TGCGGATCAG CGAGCGCAAG CTCTGGTGCT GCCGCAGGTG TTGAGTGGGT TGCAGCGTCG
AAAAGCAGCT GGTGAAGCGC TAGATATCCA AGGTGCGCGC GACACCTTGG CCAGCGCCGA
CGGCGTGCGC TTGGATCACC TGGAAATTGT CGATCCAGCC ACCCTCGAAC CATTAGAAAT
CGACGGCCTG CTCACCCAAC CAGCGTTGGT GGTCGGCGCG ATTTTCGTGG GGCCGGTGCG
 
GTTGATCGAC AATATCGAGC TCTAGTACCA ACCCTGCGTT GCAGCACGCA GCTTCGCATA
ACGCGTGCTC AGCTCAGTGT TTTTAGGTGC GCGGTGCGGA TCGGAACCGG GAGTTGGCCA
CTGCGGTGGC GTGGCCTCAC CCGACAGCGC CCATGCCGCC TGACGAGCTG CACCCAACGC
CACA 2164
(2) ANGABEN ZU SEQ ID NO: 4:
   (i) SEQUENZKENNZEICHEN:
      (A) LéNGE: 271 AminosÑuren
      (B) ART: AminosÑure
      (C) STRANGFORM: Einzelstrang
      (D) TOPOLOGIE: linear
   (ii) ART DES MOLEKöLS: Protein
   (iii) HYPOTHETISCH: NEIN
   (iv) ANTISENSE: NEIN
   (xi) SEQUENZBESCHREIBUNG: SEQ ID NO: 4: 
Met Pro Met Ser Gly Ile Asp Ala Lys Lys Ile Arg Thr Arg His
Phe 1 5 10 15

Arg Glu Ala Lys Val Asn Gly Gln Lys Val Ser Val Leu Thr Ser
Tyr
20 25 30

Asp Ala Leu Ser Ala Arg Ile Phe Asp Glu Ala Gly Val Asp Met
Leu 35 40 45

Leu Val Gly Asp Ser Ala Ala Asn Val Val Leu Gly Arg Asp Thr
Thr 50 55 60

Leu Ser Ile Thr Leu Asp Glu Met Ile Val Leu Ala Lys Ala Val
Thr 65 70 75
80

Ile Ala Thr Lys Arg Ala Leu Val Val Val Asp Leu Pro Phe Gly
Thr 85 90
95

Tyr Glu Val Ser Pro Asn Gln Ala Val Glu Ser Ala Ile Arg Val
Met 100 105 110

Arg Glu Thr Gly Ala Ala Ala Val Lys Ile Glu Gly Gly Val Glu
Ile 115 120 125

Ala Gln Thr Ile Arg Arg Ile Val Asp Ala Gly Ile Pro Val Val
 
Gly 130 135 140

His Ile Gly Tyr Thr Pro Gln Ser Glu His Ser Leu Gly Gly His
Val 145 150 155
160

Val Gln Gly Arg Gly Ala Ser Ser Gly Lys Leu Ile Ala Asp Ala
Arg 165 170
175

Ala Leu Glu Gln Ala Gly Ala Phe Ala Val Val Leu Glu Met Val
Pro 180 185 190

Ala Glu Ala Ala Arg Glu Val Thr Glu Asp Leu Ser Ile Thr Thr
Ile 195 200 205

Gly Ile Gly Ala Gly Asn Gly Thr Asp Gly Gln Val Leu Val Trp
Gln 210 215 220

Asp Ala Phe Gly Leu Asn Arg Gly Lys Lys Pro Arg Phe Val Arg
Glu 225 230 235
240

Tyr Ala Thr Leu Gly Asp Ser Leu His Asp Ala Ala Gln Ala Tyr
Ile 245 250
255

Ala Asp Ile His Ala Gly Thr Phe Pro Gly Glu Ala Glu Ser Phe
260 265 270
(2) ANGABEN ZU SEQ ID NO: 5:
   (i) SEQUENZKENNZEICHEN:
      (A) LéNGE: 279 AminosÑuren
      (B) ART: AminosÑure
      (C) STRANGFORM: Einzelstrang
      (D) TOPOLOGIE: linear
   (ii) ART DES MOLEKöLS: Protein
   (iii) HYPOTHETISCH: NEIN
   (iv) ANTISENSE: NEIN
   (xi) SEQUENZBESCHREIBUNG: SEQ ID NO: 5:

Met Gln Val Ala Thr Thr Lys Gln Ala Leu Ile Asp Ala Leu Leu
His 1 5 10
15

His Lys Ser Val Gly Leu Val Pro Thr Met Gly Ala Leu His Ser

Gly 20                    25                    30

His Ala Ser Leu Val Lys Ala Ala Arg Ala Glu Asn Asp Thr Val
    Val 35                    40                    45

Ala Ser Ile Phe Val Asn Pro Leu Gln Phe Glu Ala Leu Gly Asp
    Cys 50                    55                    60

Asp Asp Tyr Arg Asn Tyr Pro Arg Gln Leu Asp Ala Asp Leu Ala
Leu 65                    70                    75
80

Leu Glu Glu Ala Gly Val Asp Ile Val Phe Ala Pro Asp Val Glu
                    Glu 85                    90
                    95

Met Tyr Pro Gly Gly Leu Pro Leu Val Trp Ala Arg Thr Gly Ser
            Ile 100                    105                    110

Gly Thr Lys Leu Glu Gly Ala Ser Arg Pro Gly His Phe Asp Gly
        Val 115                    120                    125

Ala Thr Val Val Ala Lys Leu Phe Asn Leu Val Arg Pro Asp Arg
    Ala 130                    135                    140

Tyr Phe Gly Gln Lys Asp Ala Gln Gln Val Ala Val Ile Arg Arg
Leu 145                    150                    155
160

Val Ala Asp Leu Asp Ile Pro Val Glu Ile Arg Pro Val Pro Ile
                    Ile 165                    170
                    175

Arg Gly Ala Asp Gly Leu Ala Glu Ser Ser Arg Asn Gln Arg Leu
            Ser 180                    185                    190

Ala Asp Gln Arg Ala Gln Ala Leu Val Leu Pro Gln Val Leu Ser
        Gly 195                    200                    205

Leu Gln Arg Arg Lys Ala Ala Gly Glu Ala Leu Asp Ile Gln Gly
    Ala 210                    215                    220

Arg Asp Thr Leu Ala Ser Ala Asp Gly Val Arg Leu Asp His Leu
Glu 225                    230                    235
240

Ile Val Asp Pro Ala Thr Leu Glu Pro Leu Glu Ile Asp Gly Leu
                    Leu 245                    250
                    255

Thr Gln Pro Ala Leu Val Val Gly Ala Ile Phe Val Gly Pro Val
            Arg 260                    265                    270

Leu Ile Asp Asn Ile Glu Leu
                    275